# EUROPEAN PATENT APPLICATION

(11) **EP 3 441 758 A1**
(43) Date of publication of application: **13.02.2019**
(21) Application number: 17185968.9
(22) Date of filing: 11.08.2017
(51) Int. Cl.: G01N 33/02, A47G 21/02

(54) **SYSTEM AND APPARATUS FOR PROVIDING FOOD QUALITY INFORMATION**

(71) Applicant: ONMI B.V., 5617 BD Eindhoven (NL)
(72) Inventor: Facio Gonzales, Daniel, 5611 HL Eindhoven (Netherlands) (NL); Ayoola, Idowu, 5611 AX Eindhoven (Netherlands) (NL); Van Berlo, Sander, 5511 KW Knegsel (Netherlands) (NL); Brioschi, Luigi, 5654 NL Eindhoven (Netherlands) (NL)
(74) Representative: Herrero & Asociados, S.L.

(57) **Abstract**

Present invention refers to a system and kitchen utensil (1) for providing food quality information, where the kitchen utensil (1) comprises an elongated body with a food contact part (5) and a handle part (6); wherein the food contact part (5) comprises a temperature sensor (25), for obtaining a temperature measurement of a food; and a sodium and potassium sensor (20), for obtaining a salinity measurement at least comprising a measurement of sodium and potassium ions dissolved in the food; and wherein the handle part (6) comprises a short-range wireless communication module for providing the food quality measurements obtained by the sensors to another electronic device, which in turn provides data obtained by the sensors to a processing module implemented on an online server in communication with the smartphone (3) of the user.

## Description

### TECHNICAL FIELD OF THE INVENTION

Present invention generally relates to the field of system and apparatuses for investigating or analyzing food and more specifically to kitchen utensils and systems for providing food quality information to a user.

### BACKGROUND OF THE INVENTION

At present, a transition from traditional diets of minimally processed foods to highly processed foods and drinks is occurring. An increasing amount of people do not cook regularly or from scratch anymore and increasingly relies on heavily processed foods in their diets.

The consumption of energy-dense ultra-processed foods, unlike low-energy foods such as fruits and vegetables, promotes obesity, diabetes, long-term weight gain, cardiovascular disease and other diet-related chronic diseases, but the problem is that many people do not know where food comes from, how it is made or how it was grown.

The biggest predictor of a healthy diet is not necessarily the nutrients or calories that one is consuming, but rather whether the food is cooked by a human being. This is based on the general notions that food manufacturers use an excess of sodium and other conservatives that have a harmful effect on human health. Processed foods often contain elevated levels of sodium.

There are some solutions for measuring the salinity of food available in the prior art, which in general consist on a probe with sensors for detecting the salt concentration by analyzing its electrical conductivity of liquid and semi-liquid food to extract salt concentration data. However, the feedback provided by these probes is quite limited and the user barely receives a simple measure or a warning regarding the content of salt of his current meal.

Therefore, it is missing in the prior art a complete solution that provides the user with food quality information for its current meal, but also to help him to adopt healthy cooking routines by monitoring his daily cooking behavior.

### SUMMARY OF THE INVENTION

The present invention solves the aforementioned problems by leveraging a kitchen utensil for obtaining food quality measurements and a system for determining food quality information, mainly based on the measurements obtained by the kitchen utensil. Thus, a first aspect of present invention refers to a kitchen utensil for providing food quality measurements, the kitchen utensil comprises an elongated body with a food contact part on one end and a handle part on the other end; wherein the spoon bowl part comprises a temperature sensor, for obtaining a temperature measurement of a food within the spoon bowl part; and a sodium and potassium sensor, for obtaining a salinity measurement of the food in contact with the food contact part, wherein the salinity measurement at least comprises a measurement of sodium and potassium ions dissolved in the food; and wherein the handle part comprises a short-range wireless communication module for providing the food quality measurements obtained by the sensors to another electronic device.

Additionally, present invention may comprise at least one of: an accelerometer sensor, a gyroscope sensor and a magnetometer sensor, for obtaining motion data of the utensil. Thus, advantageously it is possible to identify cooking actions and even classifying different kind of cooking motions carried out by the user.

Additionally, present invention may comprise a sensor for measuring carbohydrate or saturated fats.

In one embodiment of the invention, it is further comprised a case for housing electrical components, being the case removable secured to the handle part of the elongated body.

According to one particular embodiment of present invention, the case comprises a plastic housing for the electrical components and a lid coupled to the plastic housing. Optionally, the case may further comprise an external protective layer (for example a silicon sleeve, a coating material or a paint), providing a splash water proof joint or heat protection between the plastic housing and the lid.

One embodiment of the present invention further comprises connecting means defining a releasable connection between the elongated body and the case. Namely, according to one particular embodiment, the connecting means comprise a first plastic hook and a second plastic hook for a physical connection between the case and the elongated body, and wherein the first plastic hook comprises a plurality of metal pins for electrical connection with the sensors embedded in the elongated body. The first hook and the second hook may be arranged on one side of the handle part of the elongated body and counterpart elements for receiving the first and the second hook may be arranged on the lid of the case, so both parts fit together.

According to one particular embodiment of present invention, the sodium and potassium sensor comprises a pair of wire electrodes connected to a Kapton film, which can be electrically connected to the short-range wireless communication module through the connecting means.

The elongated body of the kitchen utensil may be constructed of a plywood structure comprising a plurality of layers of washable wood. In one particular embodiment, the temperature sensor and the sodium and potassium sensor are embedded between the layers of washable wood.

Optionally, according to one embodiment of present invention, the case further comprises illumination means and/or vibration means for providing feedback to a user.

A second aspect of present invention refers to a system for determining food quality information comprising:
- a kitchen utensil according to any one of the embodiments disclosed before;
- a smartphone comprising:
   a short-range wireless communication module for communicating with the kitchen utensil;
   an Internet based communication module for communicating with a remote server; and
   a displaying module for displaying the food quality information; and
- a remote server configured for receiving the food quality measurements obtained by the sensors of the kitchen utensil through the Internet based communication module of the smartphone, determining food quality information based on received food quality measurements and sending the food quality information to the smartphone; wherein the food quality information at least comprises sodium content, potassium content and sodium/potassium rate.

Optionally, the system may further comprise a docking station attachable to the kitchen utensil, the docking station comprising:
- a power supply for charging the kitchen utensil;
- a short-range wireless communication module for communicating with the kitchen utensil;
- an Internet based communication module for communicating with the remote server; and
wherein the remote server is further configured for receiving the food quality measurements obtained by the sensors of the kitchen utensil through the Internet based communication module of the docking station.

Additionally, the kitchen utensil may comprise a motion sensor and the remote server is further configured for detecting and classifying cooking movements of the kitchen utensil.

In one embodiment of the present invention the short-range wireless communication module comprises a Bluetooth Smart Ready module.

Thus, at least the combination of the ion-selective salinity detection (sodium and potassium) and cooking motion detection and classification for monitoring cooking activities is unique for this application. Moreover, present invention may advantageously offer data and behavioral suggestion visualized on a smartphone, vibration/ light feedback, wireless communication with smartphone, sensor integrated into washable wood, detachable electronics case and wireless charging among other features.

Neuroscience research into mirror neurons has shown that thinking about things, or carrying out routine tasks light up the same areas of the brain. Doing something new creates a new neural pathway and that is what the solution of present invention is all about: make the users learn from their own experiences. Thus, it is possible to reconnect users with the art of cooking and stimulate them to cook with fresh and healthy ingredients. Therefore, present invention implies several advantages for users and offers a message based behavior change technology focused on the importance of a healthy diet with home cooked food instead of processed food.

### DESCRIPTION OF THE DRAWINGS

To complete the description that is being made and with the object of assisting in a better understanding of the characteristics of the invention, in accordance with a preferred example of practical embodiment thereof, accompanying said description as an integral part thereof, is a drawing wherein, by way of illustration and not restrictively, the following has been represented:
**Figure 1****.-** shows one embodiment of the kitchen utensil and docking station of present invention.
**Figure 2****.-** shows different views of the kitchen utensil and docking station of present invention.
**Figure 3****.-** shows an exploded view of the kitchen utensil of present invention.
**Figure 4****.-** shows a diagram of attachment/detachment of the body of the spatula and the handle case.
**Figure 5**.- shows an electronics block diagram of present invention.

### DETAILED DESCRIPTION OF THE INVENTION

The matters defined in this detailed description are provided to assist in a comprehensive understanding of the invention. Accordingly, those of ordinary skill in the art will recognize that changes and modifications of the embodiments described herein can be made. Also, description of well-known functions and elements are omitted for clarity and conciseness. Of course, the embodiments of the invention can be implemented in a variety of architectural platforms, operating and server systems, devices, systems, or applications. Any particular architectural layout or implementation presented herein is provided for purposes of illustration and comprehension only and is not intended to limit aspects of the invention.

Present invention discloses a kitchen utensil, namely a physical spatula, a spoon, a fork or any other food utensil/appliance able to track cooking behavior of users. Several sensors embedded in the spatula track cooking behavior of users by monitoring, for example, gestures, (in)activity or food quality based on sodium and potassium concentration. Then, the measurements taken by the spatula are processed in a server and associated to a personal profile of the user and a personalized program, which allows for context aware and responsive coaching.

The kitchen utensil of present invention measures cooking habits such as the amount of sodium and potassium used, the amount of time spent cooking and habits such as the amount of times cooked per week and the days in which a meal is cooked most often. This way, present invention deals with one of the biggest predictor of a healthy diet, which is not necessarily the nutrients or calories that you are consuming, but rather whether or not the food is cooked by a human being.

The kitchen utensil uses a salinity sensor combined with temperature and motion sensing to monitor cooking activity. The salinity sensor operates as an active selective ion detector, meaning that it receives a voltage input from a battery and uses the signal to perform impedance spectroscopy and differentiate between sodium and potassium ions dissolved in food. That is, to quantify sodium and potassium in cooking context, without having to make samples. Given the general notion that, while excesses of sodium have a harmful effect on human health, potassium represents its healthy counterpart, it was also discovered how a crucial role is played by the sodium/potassium ratio. Salinity data, in the form of sodium and potassium content and sodium/potassium ratio, is gathered continuously during food stirring/ mixing/ serving and without user input.

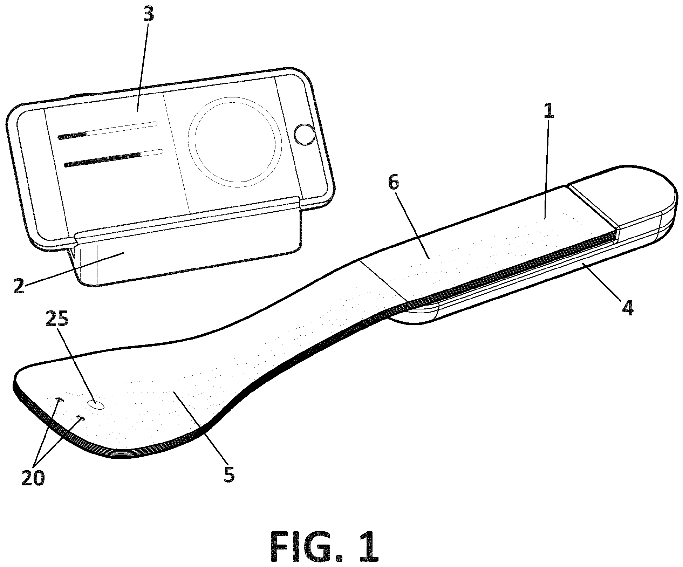

In addition to the kitchen utensil explained above, the whole system of present invention for helping users to adopt healthy cooking routines and motivate users to cook meals from scratch using fresh ingredients, according to the particular embodiment shown in figure 1, comprises four elements: the aforementioned kitchen utensil (1), a physical docking/charging station (2), an online backend server and a smartphone application (3). However, at least the docking/charging station is not an essential part of the invention, as the kitchen utensil may carry out all the communications directly with the smartphone.

The motion sensors embedded in the kitchen utensil, for example, an accelerometer, measure the general motion of the kitchen utensil. Then, integrated software is used to classify cooking motions and to measure the duration of the cooking action. Obtained data are wirelessly transferred directly the online backend server or trough the docking/charging station (2), which then transfers said data to the online backend server. Finally, the server performs analysis on the data and suggests alternative behaviors through the previously installed mobile application on the smartphone of the user when appropriate. The interpretation of salinity and motion data in combination with other patterns gives a good indication of the users' cooking routines. This allows for personalized and responsive feedback mechanisms.

The kitchen utensil uses a short-range wireless telecommunication network for directly communicating with the smartphone, for example using a low-energy Bluetooth 4.0 to transfer data to and from the mobile application installed in the smartphone of the user, or through the docking station as an interface between the kitchen utensil and the online server where the processing software resides.

On the online backend server, data are analyzed to generate behavioral suggestions that are then provided to the user on his/her smartphone via the web application. Physical feedback may also be provided through the kitchen utensil using LEDs and/or vibration.

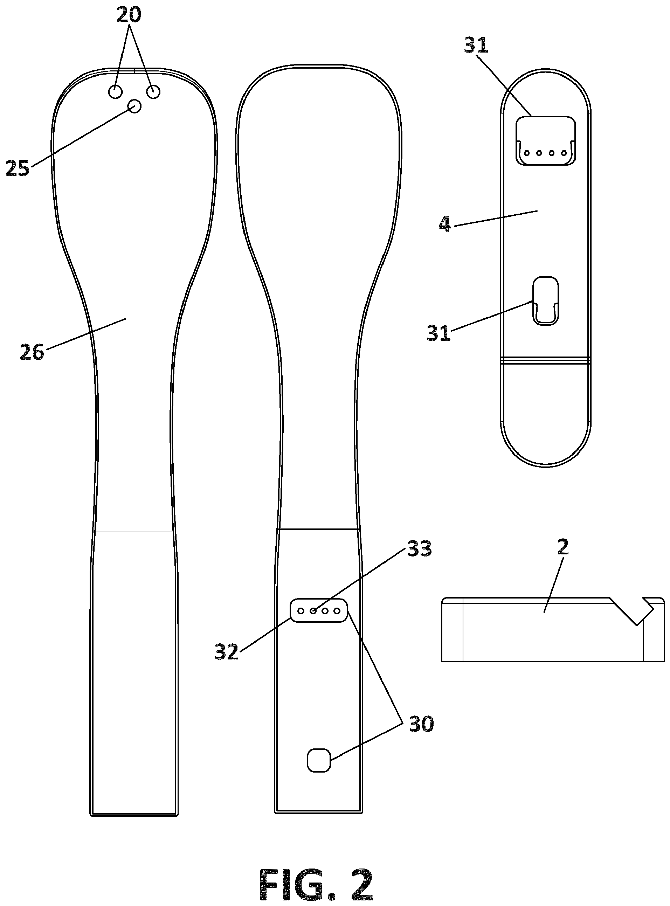

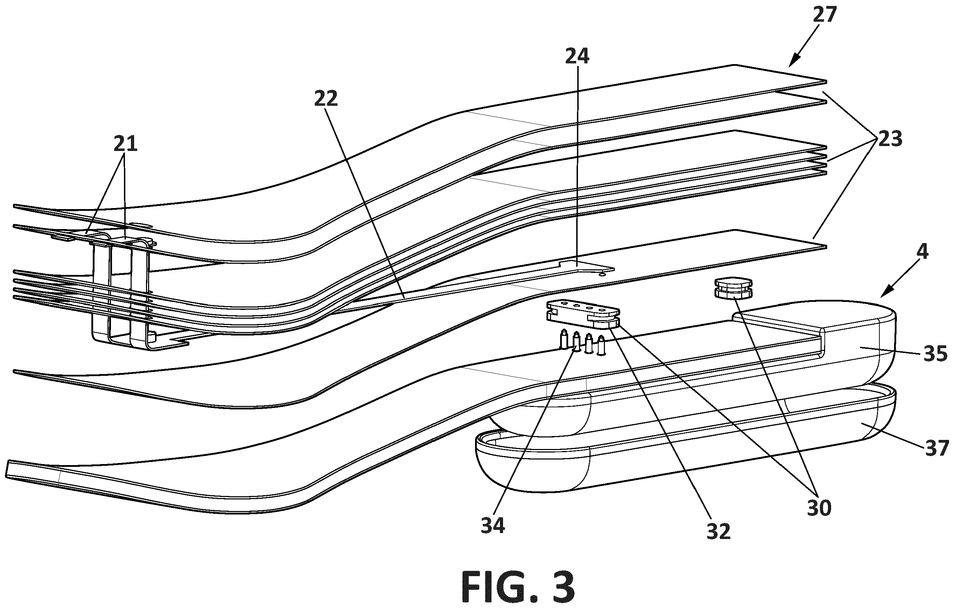

A preferred embodiment of the invention is disclosed below. Figure 2 and figure 3 show different views of the kitchen utensil (1) of present invention with details of the sodium/potassium detector (20). The sodium/potassium detector comprises a pair of thin, for example, 0.1 mm diameter, wire electrodes (21) made of a metal such as silver, for example, made of 99.9% silver. The detection of sodium and potassium is realized by means of a frequency-based technique that reveals the influence of different dissolved ions on impedance measurements. The silver wires are connected to a Kapton film (22) that is integrated between the layers of wood (23). The Kapton itself is not exposed but rather protected by the wood veneer on top. The other end of the Kapton film is exposed as a contact point (24) with the electronics within the case of the handle. The "salinity" measurements need temperature correction, so the spatula also includes in its structure a thermistor (25) arranged on the same spoon surface than the sodium/potassium detector. Namely, the thermistor has a stainless steel conductive plate that covers its sensing portion.

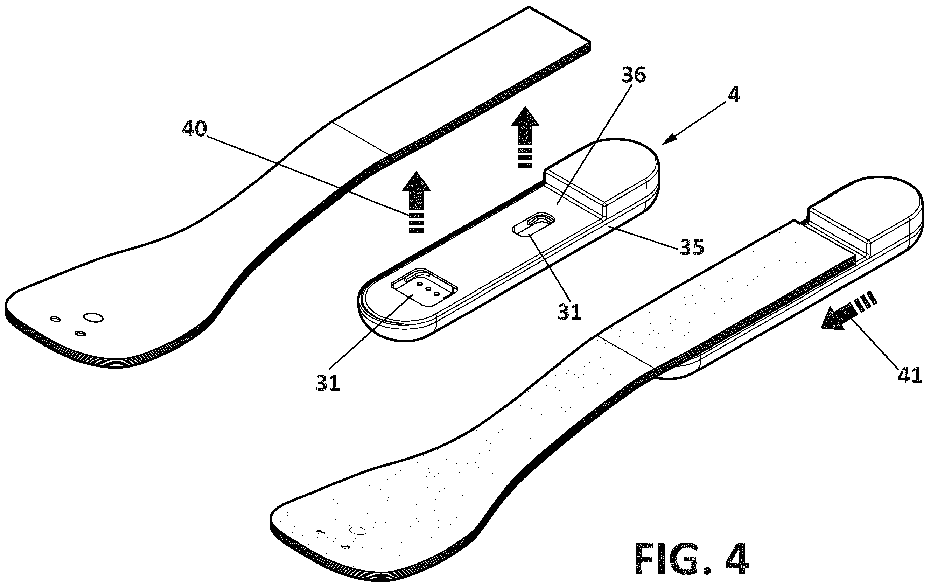

The wooden spatula (26) is the main physical (and structural) element of present embodiment. In this embodiment, it is composed of an elongated body having a food contact part (5), for example a spoon bowl shape, on one end and a handle part (6) on the other end. It is constructed of a plywood structure (27), held together with food grade FDA approved wood glue (Titebond Ultimate Wood Glue III), and with the double function of working as a traditional spatula or spoon and embedding the sensors and relative cables. In the exploded view of **figure 4** can be seen the constructive principles adopted to integrate the salinity electrodes, the temperature sensor and relative cables inside the multilayer wooden structure.

The connection between the spatula and the case is provided in the handle part of the kitchen utensil. As it can be seen in detail in **figure 3** and **figure 4****,** two plastic hooks (30) provide both physical and electrical connection between the spatula and the case, where the main electrical components are housed. Their function is to allow the detachment of the case during the hand washing of the wooden portion, which also allows easy replacement of the sensors when they corrode or damage, without having to replace the whole device. The larger (32) of the two hooks houses a plurality of metal pins (33) (four in this embodiment), that are linked to the thermistor cables and Kapton film, and that connect them to spring loaded pins (34) coming out of the case, when it is hooked to the spatula.

The case, according to this embodiment, houses the electrical components. **Figure 3** and **figure 4** illustrate the two components of the case (4), a container (35) and a lid (36). It functions also as an ergonomic handgrip for the use of the spatula during cooking activity. The case itself is a container while the lid, screwed to the case, includes the counterparts connections (31) to the physical and electrical connections installed on the elongated wooden body of the spatula. The action of attaching the case to the wooden body can be easily performed in two steps. Firstly, the user places (40) the hooks within the counterpart connections arranged on the lid of the case and then, a subtle movement (41) of the wooden spatula and the case in opposite directions make the two pieces joint. Analogously, detachment of the case can be performed by the opposite action, so the detachable sensor part can easily be replaced when worn down.

The formation of the case/lid coupling, together with an external silicon sleeve (37), provide a splash water proof joint between the two parts, that are held together by some screws (three in this embodiment) thanks to just as many threaded brass inserts.

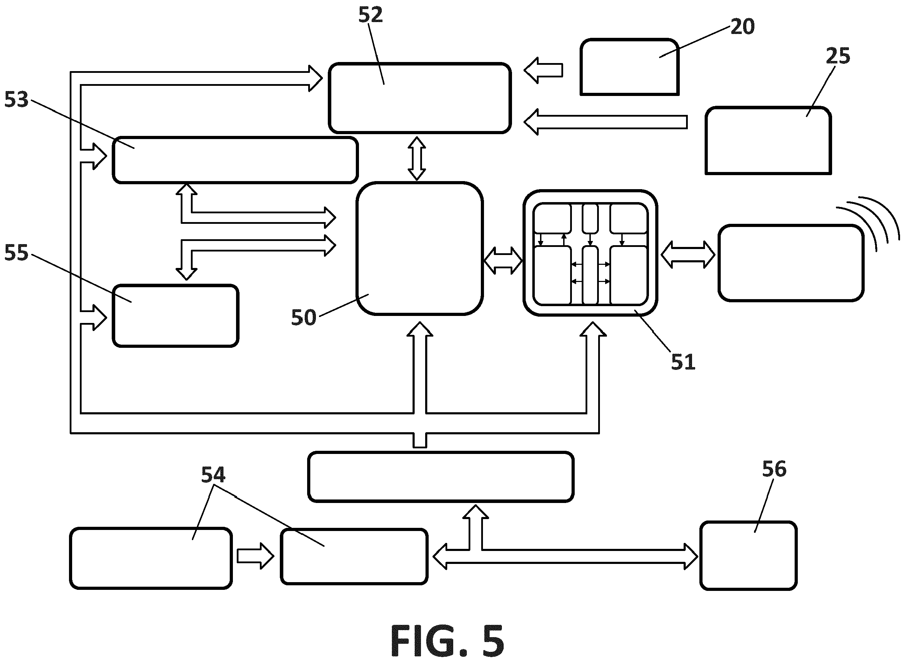

**Figure 5** shows the electronics block diagram of one embodiment of present invention, where the components comprised in the case are listed below:
- MCU (50): this embodiment uses a PIC24FJ128GB204-I/ML, which is a general Purpose 16Bit Microcontroller that works at 32MHz. It has 128KB of internal program memory and 8KB of RAM. It offers different options to interface with peripherals, like I2C, SPI, UART or USB ports, complemented with 34 general purpose Input/Outputs, in a Quad Flat-pack No-leads package.
- BT121 (51): the short-range wireless telecommunication network used in this embodiment is a *Bluetooth*® Smart Ready module targeted for applications that require both Bluetooth Smart and Classic connectivity. It can connect to legacy devices that only support Bluetooth SPP or Apple iAP2 profiles as well to devices that support Bluetooth Smart.
- Signal Conditioning Circuit (52): this is based on two different components, a Digital-to-Analog Converter (for example AD5541 AARMZ) and an Analog-to-Digital converter (for example ADS8320E/250):
   ▪ *AD5541AARMZ* from Analog-Devices is a single 16-bit serial input unbuffered voltage output Digital-to-Analog Converter (DAC). The DAC output range extends from 0V to VREF and is guaranteed monotonic, providing ±1LSB INL accuracy at 16-bit without adjustment over the full specified temperature range of -40 to 125°C. Offering unbuffered outputs, the AD5541A achieves a 1µs settling time with low power consumption and low offset errors. Provide low noise performance of 11.8nV/√Hz and low glitch. It uses a versatile 3-wire interface that is compatible with 50MHz SPI, QSPI™, MICROWIRE™ and DSP interface standards.
   ▪ *ADS8320E*/*250* from Texas Instruments is a 16-bit sampling Analog-to-Digital Converter (ADC) with ensured specifications over a 2.7 to 5.25V supply range. It requires very little power even when operating at the full 100kHz data rate. At lower data rates, the high speed of the device enables it to spend most of its time in the power-down mode - the average power dissipation is less than 100µW at 10kHz data rate. The ADC also features a synchronous serial (SPI/SSI compatible) interface and a differential input. Ultra-low power and small size make the ADC ideal for portable and battery-operated systems. It is also a perfect fit for remote data acquisition modules, simultaneous multi-channel systems, and isolated data acquisition.
- Accelerometer-Gyroscope-Magnetometer **(53):** that is, a motion data unit, which in this particular embodiment is a LSM9DS0, a system-in-package featuring a 3D digital linear acceleration sensor, a 3D digital angular rate sensor, and a 3D digital magnetic sensor. The motion data unit LSM9DS0 has a linear acceleration full scale of ±2g/±4g/±6g/±8g/±16g, a magnetic field full scale of ±2/±4/±8/±12 gauss and an angular rate of ±245/±500/±2000 dps. It includes an I2C serial bus interface supporting standard and fast mode (100 kHz and 400 kHz) and an SPI serial standard interface. Magnetic, accelerometer and gyroscope sensing can be enabled or set in power-down mode separately for smart power management.
- Charging port **(54):** this embodiment of present invention uses a wireless interface based on a magnetic induction method to charge the battery. This is the Qi standard interface for the inductive electrical power transfer developed by the Wireless Power Consortium. The Qi system comprises a power transmission pad (the docking station) and a compatible receiver in a portable device and can transfer electrical power over distances up to 7mm. Present invention integrates the bq51013 wireless power supply receiver from Texas Instruments, an advanced, integrated, receiver IC for wireless power transfer in portable applications. The device provides the AC/DC power conversion while integrating the digital control required to comply with the Qi v1.0 communication protocol. Together with a transmitter controller, the bq51013 enables a complete contact-less power transfer system for a wireless power supply solution.
- SD card (SanDisk) and socket **(55):** a small sized flash memory used to store large data locally for up to 8GB.
- A battery **(56):** a rechargeable 3.6 V, 150 mAh nickel metal hydride button cell with PCB terminal pins and with protection circuit. A step-down regulator is included, and the device runs on a 3V supply. The battery operates safely at high temperatures.
- Charging coil: the receiving end of the induction (wireless) charging device for the battery.
- Physical feedback devices: according to different embodiments, the kitchen utensil comprises a series of LEDs and a vibration motor to provide the user with real time feedback.

The base or docking station (2) complements the kitchen utensil and, in addition to its function as a charging port, the docking station may play the role of exchanging information with the kitchen utensil via the short-range wireless communication network, for example, BLE, and establishing another communication path between the kitchen utensil and the online server via Wi-Fi (or Ethernet) connection. The docking station, according to one particular embodiment, comprises a microcontroller, for example a "Raspberry Pi 3" that handles all the functions of the base (for example charging or BLE communication with the device and internet connection to the server); a charging coil, namely the transmitter coil of the inductive charging system; one or more LEDs for providing feedback on the status of the spatula (like Bluetooth connection or charging) and the base itself (like power or internet connection); and a power input.

## Claims

1. A kitchen utensil for providing food quality measurements **characterized by** comprising an elongated body (1) with a food contact part (5) on one end and a handle part (6) on the other end;
wherein the food contact part comprises a temperature sensor (25), for obtaining a temperature measurement of a food in contact with the food contact part; and a sodium and potassium sensor (20), for obtaining a salinity measurement of the food in contact with the food contact part, wherein the salinity measurement at least comprises a measurement of sodium and potassium ions dissolved in the food; and
wherein the handle part comprises a short-range wireless communication module for providing the food quality measurements obtained by the sensors to another electronic device.

2. Utensil according to claim 1 further comprising at least one of: an accelerometer sensor, a gyroscope sensor and a magnetometer sensor, for obtaining motion data of the utensil.

3. Utensil according to any one of previous claims further comprising a case (4) for housing electrical components, being the case removable secured to the handle part of the elongated body.

4. Utensil according to claim 3, wherein the case comprises a plastic housing (35) for the electrical components and a lid (36) coupled to the plastic housing,

5. Utensil according to claim 4 wherein the case further comprises an external protective layer (37) providing a splash water proof joint and/or heat resistance between the plastic housing and the lid.

6. Utensil according to any one of claims 3-5, further comprising connecting means defining a releasable connection between the elongated body and the case.

7. Utensil according to claim 6, wherein the connecting means comprise a first plastic hook (32) and a second plastic hook (30) for a physical connection between the case and the elongated body, and wherein the first plastic hook comprises a plurality of metal pins (33) for an electrical connection with the sensors embedded in the elongated body.

8. Utensil according to claim 7 wherein the first hook and the second hook are arranged on one side of the handle part of the elongated body and counterpart elements (31) for receiving the first and the second hook are arranged on the lid of the case.

9. Utensil according to any one of claims 6 - 8, wherein the sodium and potassium sensor comprises a pair of wire electrodes (21) connected to a Kapton film (22), which can be electrically connected to the short-range wireless communication module through the connecting means.

10. Utensil according to any one of previous claims wherein the elongated body is constructed of a plywood structure comprising a plurality of layers (23) of washable wood.

11. Utensil according to claim 10 wherein the temperature sensor and the sodium and potassium sensor are embedded between the layers of washable wood.

12. Utensil according to any one of previous claims, wherein the case further comprises illumination means and/or vibration means for providing feedback to a user.

13. System for determining food quality information comprising:
- a kitchen utensil according to any one of previous claims;
- a smartphone comprising:
a short-range wireless communication module for communicating with the kitchen utensil;
an Internet based communication module for communicating with a remote server;
a displaying module for displaying the food quality information; and
- a remote server configured for receiving the food quality measurements, obtained by the sensors of the kitchen utensil, through the Internet based communication module of the smartphone, determining food quality information based on received food quality measurements and sending the food quality information to the smartphone; wherein the food quality information at least comprises sodium content, potassium content, and sodium/potassium rate.

14. System according to claim 13 further comprising a docking station (2) attachable to the kitchen utensil, the docking station comprising:
- a power supply for charging the kitchen utensil;
- a short-range wireless communication module for communicating with the kitchen utensil;
- an Internet based communication module for communicating with the remote server; and
wherein the remote server is further configured for receiving the food quality measurements, obtained by the sensors of the kitchen utensil, through the Internet based communication module of the docking station.

15. System according to any one of claims 13-14, wherein the kitchen utensil comprises a motion sensor and the remote server is further configured for detecting and classifying cooking movements of the kitchen utensil.
